# EUROPEAN PATENT SPECIFICATION

(11) **EP 2 238 939 B1**
(45) Date of publication and mention of the grant of the patent: **28.03.2012**
(21) Application number: 10154276.9
(22) Date of filing: 22.02.2010
(51) Int. Cl.: A61B 18/20, A61B 17/225

(54) **Control and support system for blemish treatment apparatus**
Steuerungs- und Unterstützungssystem für Fehlerbehandlungsvorrichtung
Système de commande et de support pour appareil de traitement de tâches

(30) Priority: 31.03.2009 IT MI20090510
(43) Date of publication of application: 13.10.2010
(73) Proprietor: Novavision Group S.r.l, 20123 Milano (IT)
(72) Inventor: Crapelli, Danilo, I-20123 Milano (IT)
(74) Representative: Cicogna, Franco

(56) References cited:
- DE-A1- 4 120 074
- US-A- 5 330 517
- US-A- 5 586 981
- US-A- 5 827 204
- US-A- 5 879 346
- US-B1- 6 350 245
- US-B1- 6 676 654

## Description

### BACKGROUND OF THE INVENTION

The present invention relates to a control and support system particularly for blemish treatment, apparatus.

As is known, for treating blemishes both in the aesthetic and medical fields, technological sound, cavity, RF processing apparatus are conventionally used.

In using the above technological apparatus, the achieved results and operating safety strictly depend on the skillness of the apparatus operator.

In fact, the above apparatus usually require a proper locating by the operator of the apparatus handle on the region to be processed, to allow the operator to move the apparatus for the overall duration of the treatment.

This operating method, which depends on the operator selection, does not allow to achieve a homogeneous treatment through the overall region to be treated, which, moreover, could represent a danger if the operator would stop for a long time on a single specific skin region.

Document US-B1-6 676 654 discloses a control and support system for a skin blemish treatment apparatus, the system comprising: indicating means, adapted for signaling or drawing, on a person to be treated, an skin region to be treated in an active operation of said apparatus; while indicating a position thereat an operator must locate and hold an apparatus handle.

Document US-A-5 330 517 discloses a device for treatment of tissue, the device comprising a laser provided with means enabling it to emit laser radiation in the form of a sufficient amount of pulses to deliver a total energy to the heat treatment of the tissue, the device being characterized in that it includes means for adjusting the number and the duration of the pulses.

### SUMMARY OF THE INVENTION

Accordingly, the aim of the present invention is to provide a control system adapted to properly support a medical apparatus for treating skin blemishes.

Within the scope of the above mentioned aim, a main object of the invention is to provide such a control system allowing to safely control the operator in performing homogeneous and efficient skin blemish treatment operations.

Another object of the present invention is to provide such a control system which, owing to its specifically designed construction and features, is adapted to provide a very safe and reliable operation of the blemish treatment or processing apparatus.

According to one aspect of the present invention, the above mentioned aim and objects, as well as yet other objects, which will become more apparent hereinafter, are achieved by a control and supporting system and a skin blemish treatment apparatus, according to claim 1.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative example, in the accompanying drawings, where:

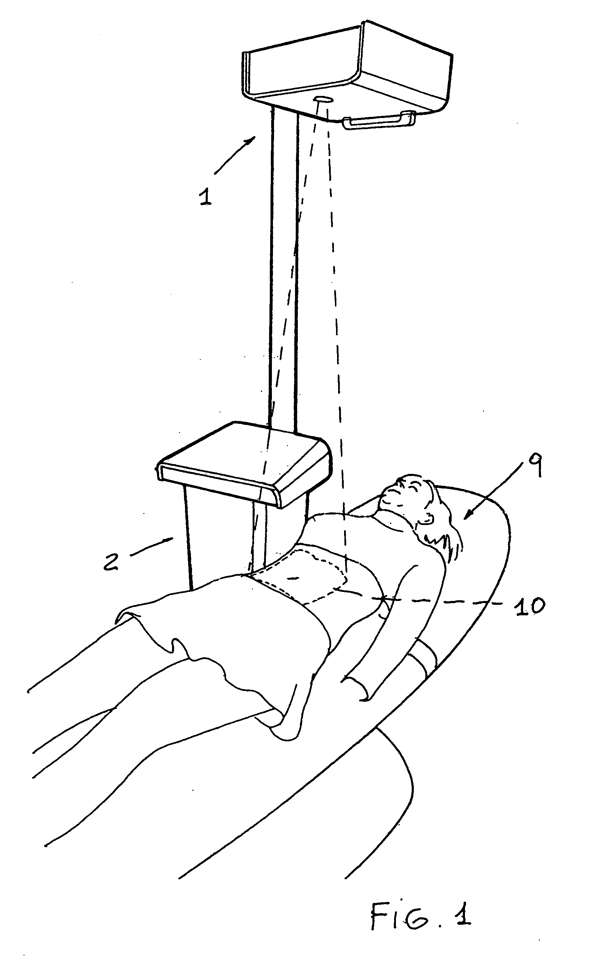

Figure 1 is a perspective view showing a control system according to the present invention applied to a generic apparatus to be used in a medical or aesthetic field;

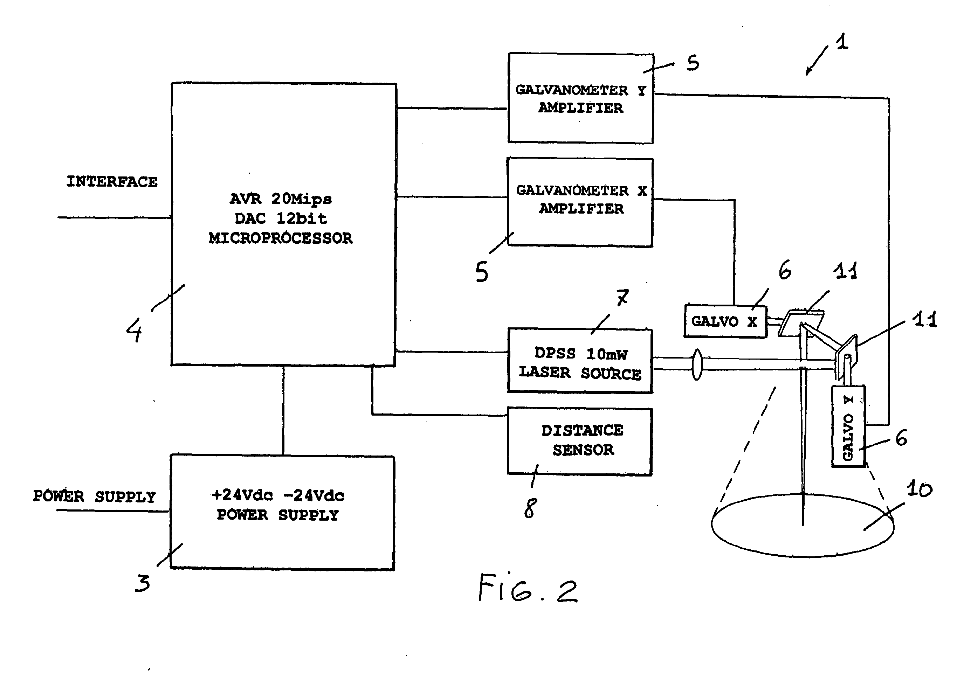

Figure 2 shows a block diagram of the control system according to the present invention;

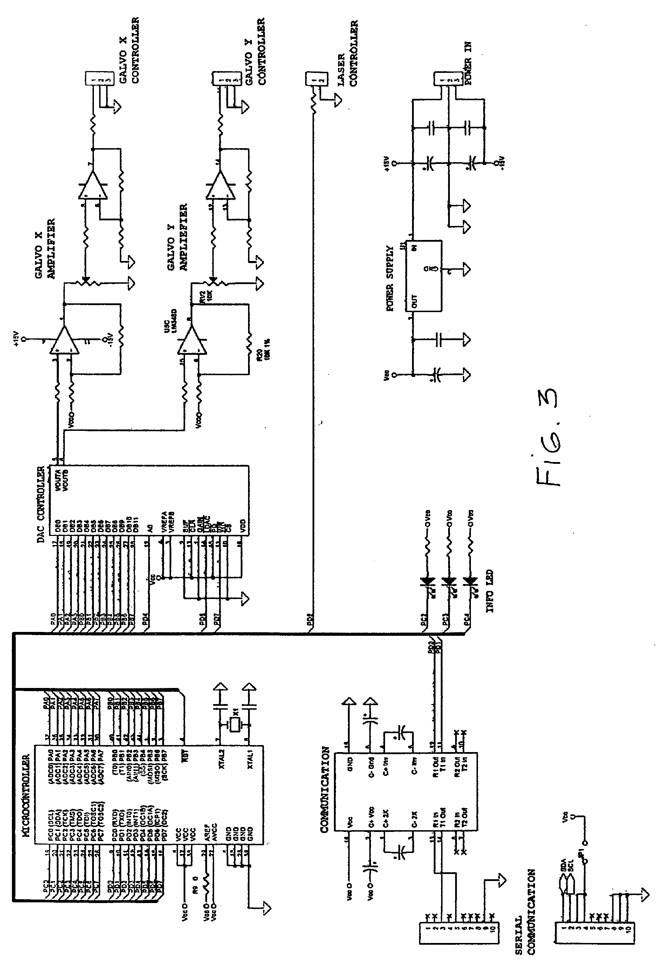

Figure 3 shows an electric diagram of the control system according to the present invention; and
Figure 4 schematically shows the operation of mirror galvanometers used for deflecting a laser beam in the control system according to the present invention.

### DESCRIPTION OF THE PREFERRED EMBODIMENTS

With reference to the number references of the above mentioned figures, the control and support system according to the present invention, which has been generally indicated by the reference number 1, comprises a box-like body applied to a generic apparatus 2 for medical or aesthetic use.

More specifically, the control system according to the present invention substantially comprises a system net power supply 3, with a DC 24 V +/- output, a microprocessor system 4, for receiving and processing data and controlling the galvanometers and laser, a power amplifier card or board 5, for controlling each said galvanometer 6, a laser source 7 and a distance sensor 8.

The control system 1 has been specifically designed to allow the operator to be properly and precisely driven to a skin region to be processed or treated.

In fact, the inventive system indicates on the person 9 or patient to be treated, a region 10 thereat the operator must perform the blemish treatment process.

In particular, during the treatment time, the system 1 indicates to the operator where he/she must arrange the apparatus active handle, as well as the time through which the operator must leave the apparatus handle at that region.

According to one aspect of the present invention, the control system according to the present invention further comprises a software control auxiliary system which, as properly programmed depending on the apparatus to be used, will send data to the control system proper, said data indicating to the operator the time through which said operator must leave the apparatus at a given skin region.

Said auxiliary system is set by the operator by supplying to said auxiliary system the physical parameters of the person to be treated, upon which a computer will send back, based on pre-stored formulas, the proper time and position for performing an efficient and safe treatment operation.

The control system technology is based on an electronic system adapted to signal, or "draw or pattern" on the person being treated, the involved skin region, during an active treatment process, while indicating the position thereat the apparatus operator must arrange the apparatus handle.

In particular, the control system according to the present invention has been designed by using a low emission laser source, preferable a 10 mW laser source, the laser beam of which is reflected by two reflecting mirrors 11 coupled to respective precision galvanometers 6.

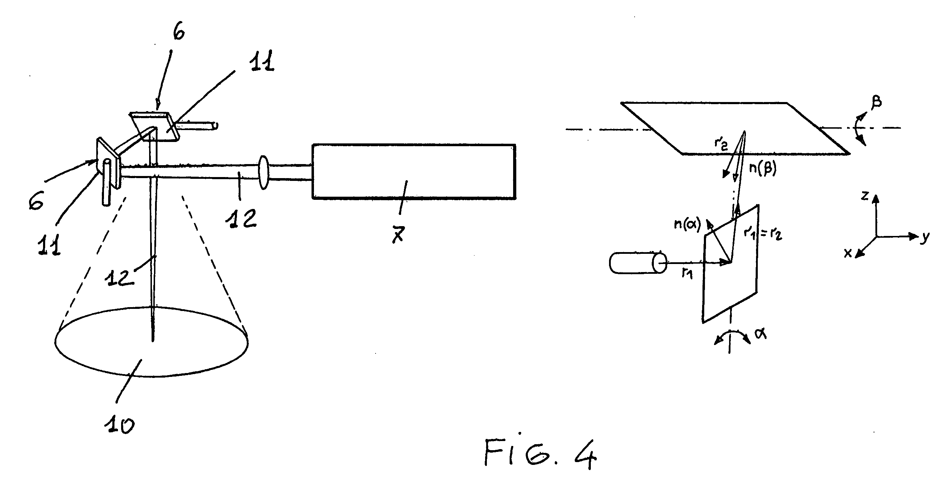

As is schematically shown in figure 4, said mirrors 11, mounted according to coordinates X and Y, allow the laser beam 12 to be deflected with a very high deflecting speed, thereby drawing or patterning any desired shapes or patterns on the person being treated, to also indicate, during the treatment, a precise point or spot thereat the operator must arranged the apparatus handle.

Said galvanometers 6 are controlled by the microprocessor system 4 which is coupled, through a communicating port (RS232, a parallel, USB, wireless port and so on) to the treatment apparatus 2, which will send to the control system 1 the data required for drawing the region to be treated 10 and the proper position of the apparatus handle.

The control system 1 comprises moreover a component 8 for detecting the distance between the laser source and the person to be treated.

Said sensor preferably comprises a ultrasound distance sensor; however, it would be also possible to use other types of sensors, such as laser, IR, mechanical sensors and so on, for detecting said distance.

Such an arrangement will allow the control system 1 to autonomously recalculate the laser reflecting parameters to in turn allow the operator to achieve in any conditions a constant locating and sizing parameter arrangement.

The galvanometer used in the inventive system is an instrument adapted to transform an electric current into a mechanical torque.

Actually, a galvanometer suitable to deflect a mirror is used in laser systems for properly directing the laser beam.

Preferably, a high power galvanometer system, constituting part of a feedback servosystem, with a very quick and precise frequency response is herein used.

The inventive system, as stated, comprises two galvanometers 6 with related mirrors 11 adapted to deflect the laser beam 12 from the laser source 7 and to project said laser beam on the person to be treated 9, thereby drawing any desired pattern 10 thereon, or indicating, through a point or spot, a proper locating of the apparatus handle, during the treatment time.

As stated, said galvanometers are controlled by the dedicated microprocessor system 4.

On the other hand, the auxiliary system comprises a specifically designed software which has been custom designed depending on the apparatus used for aiding the operator during the treatment, so as to indicate to said operator, through the control system, a proper treatment time as well as a related rest time at a given region to be treated.

Actually, the above auxiliary system together with the inventive control system, allows, by specifically setting the physical parameters of the person to be treated by the operator, to indicate the overall treatment time, and the location of the apparatus handle on the region to be treated as well as the rest time for each said region to be treated.

The auxiliary system, in particular, will send the above information to the control system which, in turn, will clearly display on the subject to be treated the handle locating time to be followed by the operator.

An application example of the control system according to the present invention, as applied to a cavitational time of treatment apparatus will be briefly disclosed hereinbelow.

As is known, cavitation is a phenomenon consisting of generating steam regions or bubbles in a liquid, which successively will implode.

This is due to a local pressure decrease to a value less than the vapor pressure or tension of said liquid, which is compelled to be subjected to a phase change to a gas status, thereby forming steam or vapor containing cavities.

The steam or vapor containing cavities will be held as such up to their exit from the low hydrostatic pressure region: as they return to a rest fluid region, the vapor pressure will not be sufficient to overcome the hydrostatic pressure and the cavitation "bubbles" will abruptly implode thereby emitting energy.

The ultrasound vibrations generate wave through the liquid which waves, by alternately providing a pressing step and a releasing step, produce a very high turbulence and associated cavitation bubbles which, by imploding, develop a very high pressure.

As greater is the pressing and releasing action, as higher is the provided energy.

The above cavitation phenomenon, as is well known to one skilled in the art, is used in aesthetic medical fields for reducing and reshaping fat tissue having adipocytes as its morpho-functional units.

Said adipocytes contain variable fat amounts, and frequently form fat pools, split into lobules by connective sediments where blood vessels are arranged.

Said cavitation allows excess fats and cellulitis blemishes to be controllably reduced through a non invasive short and safe treatment.

The impact wave generated by the "bubble" implosion in the fat tissue aids liquid drains and releases of the fat acids from the cells, with a great reduction of the excess volume.

The interaction with the tissues is of a mechanical and thermal type.

The mechanical effect is a movement of the tissue particles therethrough ultrasounds pass, whereas the thermal effect is a direct consequence of the mechanical one, since heat is generated by vibration, impact and friction of the cellular and intracellular structures forming the tissues therethrough ultrasounds pass.

Such a temperature increase both occurs on the surface and at deep regions of the tissue being treated and is a very quick one.

Said cavitational systems may be advantageously aided by the control system according to the present invention.

Actually, the operator sets, by the cavitational apparatus, the region to be treated, while the control system 1 projects on the person 9 to be processed a geometric pattern 10 delimiting the active region.

In particular, said operator sets, depending on the region to be processed and processing method, the treatment overall time and, by actuating the apparatus, the control system will indicate by a point or spot that region at which the ultrasound emitting handle must be located and driven.

After a passage of a partial treatment time, the system will switch off the emission of ultrasounds and the indicating point or spot.

Then, the control system will indicate a second point or spot to be treated, and will switch on again the laser source and ultrasound emitter, and this procedure will be continued up to the end of the treatment.

Thus, with the above disclosed system the set region to be treated is homogeneously and efficiently processed.

As stated, the above cavitational system may be advantageously aided by the control and auxiliary systems according to the present invention.

To that end, the operator will set, from the cavitational apparatus, the region to be treated, and the control system will project on the person 9 to be treated the geometrical shape 10 delimiting the active region.

In particular, the operator sets the physical parameters of the person to be treated, such as a thickness, a treatment time for each region, and so on, thereby the system will recalculate, through the auxiliary system stored formulas, the overall treatment time, the number of the regions to be treated, as well as the safe time for each said region.

Upon switching on the apparatus, the control system will indicate by an indicating point or spot the region thereat the ultrasound emitting handle must be arranged and driven through.

At the end of the treatment time for each region, determined by the auxiliary system, the inventive system will switch off both the ultrasound emitter and the indicating point or spot.

Then, said control system 1 will indicate or display a second point or spot to be treated, while switching on again the laser source and ultrasound emitter, and this procedure will continue up to the end of the target treatment.

With the above disclosed system, it is possible to perform a homogeneous, efficient and safe treatment through the overall set region or zone to be treated.

It has been found that the invention fully achieves the intended aim and objects.

In fact, the invention has provided a control system which may be applied to different types of technologic apparatus, operating either based on sound, cavitation, or RF methods and so on, said system safely and precisely indicating to the operator where arranging the apparatus active handle, and further indicating the apparatus rest time at that given or target region.

In practicing the invention, the used materials, as well as the contingent size and shapes, can be any, according to requirements.

## Claims

1. A control and support system (1) and a skin blemish treatment apparatus (2) comprising a treatment handle,
- said control and support system comprising,
- a laser source (7) for emitting a laser beam and means (4,6,11) for controlling and positioning the laser beam of said laser source,
- means (4) for receiving and processing, from said operator physical parameters of the person to be treated, such as skin thickness and processing time per region, wherefrom said means for processing calculates, through formulas stored in said control and support system, an overall treatment time, the number of regions to be treated and a safety time for each region,
- said control and support system (1) being adapted to cause said laser source (7) and to cause said means (4,5,6,11) for controlling said laser beam, in an active operation of said treatment apparatus,
- to project, on a person to be treated, a geometric pattern delimiting a treatment region (10),
- to indicate a position whereat an operator must locate said treatment handle, and
- to indicate a precise rest time for which time the operator must hold said treatment handle at the indicated treatment location,
whereby, upon switching on the treatment apparatus (2), the control and support system (1) will cause said means (4,5,6,11) for controlling and positioning the laser beam to indicate a point or spot whereat said treatment handle must be located and, after said rest time having passed, said control system switching off said treatment apparatus (2) and said laser beam, and then said control system causes said laser to indicate a second point or spot to be treated by switching on again said laser source (7) and said treatment apparatus (2), this procedure being continued until to the end of the treatment time.

2. A control system, according to claim 1, **characterized in that** said control system (1) comprises an auxiliary system designed for indicating to the operator, during the treatment, a proper treatment time and a precise rest time at a set skin region.

3. A control system, according to claim 1, **characterized in that** said control system (1) comprises a net power supply (3), providing a DC +/-24 V output, a microprocessor system (4) for receiving and processing data and controlling galvanometers (6) and a laser source (7), a power amplifier board (5), for controlling each of said galvanometers, a laser source (7) and a distance sensor (8).

4. A control system, according to claim 1, **characterized in that** said control system (1) comprises a low emission laser source emitting a laser beam which is reflected by two mirrors (11) coupled to corresponding precision said galvanometers (6), said mirrors (11) being mounted on coordinates X and Y and allowing to deviate the laser beam (12) generated by said laser source, with a very high speed thereby drawing or patterning any desired types of pattern on the person (9) to be treated while indicating, during the treatment, a point or spot thereat the operator must arrange the apparatus handle, said galvanometers (6) being controlled by a microprocessor system (4) which is connected through a treatment apparatus communicating port, said treatment apparatus sending data to be used by the control system to pattern regions to be treated and to consequently locate the apparatus handle.

5. A control system, according to claim 4, **characterized in that** said distance sensor (8) for detecting the distance from the laser source (7) to the person (9) to be treated, is preferably constituted by an ultrasound distance sensor (8), a laser sensor, a IR sensor, a mechanical sensor, to allow the control system to autonomously recalculate the laser reflecting parameters to always provide a constant or even locating and sizing of said handle.

6. A control system, according to claim 2, **characterized in that** said auxiliary system (1) is set by the operator through physical parameters of the person (9) to be treated, thereby a computer, based on prestored formulas, calculates optimal times and positions to provide an efficient and safe treatment of said person.

7. A control system, according to claim 2, **characterized in that** said auxiliary system, in cooperation with said control system (1), allows, by setting said physical parameters of the person (9) to be treated, to indicate a proper overall treatment time, a location of the apparatus handle at the skin region (10) to be treated, and a rest time of said handle for each skin region to be treated, said auxiliary system sending this information to the control system (1) in turn precisely displaying on the person (9) to be treated the operating time and the apparatus handle location to be followed by the operator.

8. A control system, according to claim 2, **characterized in that**, with said control system applied to a cavitational treatment apparatus, in cooperation with said auxiliary system, said operator sets from said cavitational apparatus a skin region to be treated thereby said control system will project on the person to be treated a geometric pattern delimiting an active region, the operator further setting said physical parameters of the person to be treated, such as a thickness, processing time per region, thereby said control system will recalculate, through formulas stored in said auxiliary system, an overall treatment time, a number of regions to be treated and a safety time for each region, and, upon switching off the apparatus, said control system indicating by a point or spot a region thereat arranging and driving the ultrasound emitter handle, and, after having treated said region for a set treatment time, set by said auxiliary system, said control system switching off the ultrasound emitter and indicating point or spot, and then the control system indicating a second point to be treated while switching on again said laser source and ultrasound emitter, the disclosed procedure being continued up to the end of the treatment.

## Patentansprüche

1. Steuerungs- und Stützsystem (1) und eine Vorrichtung (2) zur Behandlung von Hautunreinheiten, die einen Behandlungsgriff umfasst,
- wobei das Steuerungs- und Stützsystem folgendes umfasst:
- eine Laserquelle (7) zum Emittieren eines Laserstrahls und Mittel (4, 6, 11) zum Steuern und Positionieren des Laserstrahls der Laserquelle,
- Mittel (4) zum Aufnehmen und Verarbeiten von physischen Parametern der Person, die behandelt werden soll, wie zum Beispiel Hautdicke und Verarbeitungszeit pro Bereich, durch den Operator, woraus das Mittel zum Verarbeiten über Formeln, die im Steuerungs- und Stützsystem gespeichert sind, eine Gesamtbehandlungszeit, die Zahl der Bereiche, die behandelt werden sollen, und eine Sicherheitszeit für jeden Bereich berechnet,
- wobei das Steuerungs- und Stützsystem (1) so angepasst ist, dass es die Laserquelle (7) und das Mittel (4, 5, 6, 11) zum Steuern des Laserstrahls bei der aktiven Arbeit der Behandlungsvorrichtung steuert,
- um auf eine Person, die behandelt werden soll, ein geometrisches Muster zu projizieren, das einen Behandlungsbereich (10) abgrenzt,
- um eine Position anzuzeigen, auf welche ein Operator den Behandlungsgriff platzieren muss, und
- um eine genaue Restzeit anzuzeigen, während der der Operator den Behandlungsgriff am angezeigten Behandlungsort halten muss,
wodurch beim Einschalten der Behandlungsvorrichtung (2) das Steuerungs- und Stützsystem (1) bewirkt, dass das Mittel (4, 5, 6, 11) zum Steuern und Positionieren des Laserstrahls einen Punkt oder einen Fleck anzeigt, an der der Behandlungsgriff platziert werden muss, und, nachdem die Restzeit abgelaufen ist, das Steuerungssystem die Behandlungsvorrichtung (2) und den Laserstrahl abschaltet, und dann das Steuerungssystem bewirkt, dass der Laser einen zweiten Punkt oder Fleck anzeigt, die behandelt werden soll, wobei die Laserquelle (7) und die Behandlungsvorrichtung (2) wieder eingeschaltet werden, wobei diese Prozedur bis zum Ende der Behandlungszeit fortgesetzt ist.

2. Steuerungssystem nach Anspruch 1, **dadurch gekennzeichnet, dass** das Steuerungssystem (1) ein Hilfssystem umfasst, das während der Behandlung zum Anzeigen einer korrekten Behandlungszeit und einer genauen Restzeit in einem eingestellten Hautbereich für den Operator ausgelegt ist.

3. Steuerungssystem nach Anspruch 1, **dadurch gekennzeichnet, dass** das Steuerungssystem (1) eine Netzstromversorgung (3), die für eine +/- 24 V DC-Ausgabe sorgt, ein Mikroprozessorsystem (4) zum Aufnehmen und Verarbeiten von Daten und zum Steuern von Galvanometern (6) und einer Laserquelle (7), eine Leistungsverstärkerplatine (5) zum Steuern jedes der Galvanometer, einer Laserquelle (7) und eines Abstandssensors (8) umfasst.

4. Steuerungssystem nach Anspruch 1, **dadurch gekennzeichnet, dass** das Steuerungssystem (1) eine emissionsarme Laserquelle umfasst, die einen Laserstrahl emittiert, welcher von zwei Spiegeln (11) reflektiert ist, die mit entsprechenden Präzisionsgalvanometern (6) verbunden sind, wobei die Spiegel (11) auf den Koordinaten X und Y befestigt sind und ermöglichen, dass der Laserstrahl (12), der von der Laserquelle erzeugt ist, mit einer sehr hohen Geschwindigkeit abgelenkt wird, wodurch jede gewünschte Art von Mustern auf der Person (9), die behandelt werden soll, gezeichnet oder gestaltet wird, während gleichzeitig mit der Behandlung ein Punkt oder ein Fleck angezeigt ist, auf welcher der Operator den Behandlungsgriff anordnen muss, wobei die Galvanometer (6) durch ein Mikroprozessorsystem (4) gesteuert sind, das durch einen Kommunikationsport der Behandlungsvorrichtung angeschlossen ist, wobei die Behandlungsvorrichtung Daten sendet, die vom Steuerungssystem verwendet werden sollen, um die Bereiche, die behandelt werden sollen, mit einem Muster zu versehen und folglich den Behandlungsgriff zu platzieren.

5. Steuerungssystem nach Anspruch 4, **dadurch gekennzeichnet, dass** der Abstandssensor (8) zum Feststellen des Abstandes zwischen der Laserquelle (7) und der Person (9), die behandelt werden soll, vorzugsweise aus einem Ultraschallabstandssensor (8), einem Lasersensor, einem IR-Sensor, einem mechanischen Sensor aufgebaut ist, so dass das Steuerungssystem autonom die Laserreflektionsparameter neu berechnen kann, um immer zu erreichen, dass der Behandlungsriff konstant platziert und bemessen ist.

6. Steuerungssystem nach Anspruch 2, **dadurch gekennzeichnet, dass** das Hilfssystem (1) vom Operator durch physische Parameter der Person (9), die behandelt werden soll, eingestellt ist, wodurch ein Computer auf der Grundlage von vorgespeicherten Formeln optimale Zeiten und Positionen berechnet, um für eine effiziente und sichere Behandlung der Person zu sorgen.

7. Steuerungssystem nach Anspruch 2, **dadurch gekennzeichnet, dass** das Hilfssystem in Kooperation mit dem Steuerungssystem (1) durch Einstellen von physischen Parametern der Person (9), die behandelt werden soll, eine korrekte Gesamtbehandlungszeit, einen Ort für den Vorrichtungsgriff auf dem Hautbereich (10), der behandelt werden soll, und eine Restzeit des Griffs für jeden Hautbereich, der behandelt werden soll, anzeigen kann, wobei das Hilfssystem diese Informationen an das Steuerungssystem (1) sendet, das wiederum an der Person (9), die behandelt werden soll, genau die Betriebszeit und die Lage des Vorrichtungsgriffs anzeigt, die vom Operator zu beachten sind.

8. Steuerungssystem nach Anspruch 2, **dadurch gekennzeichnet, dass** bei dem Steuerungssystem, das auf eine Vorrichtung zur Kavitationsbehandlung angewendet ist, in Kooperation mit dem Hilfssystem der Operator an der Kavitationsvorrichtung einen Hautbereich einstellt, der behandelt werden soll, wodurch das Steuerungssystem auf die Person, die behandelt werden soll, ein geometrisches Muster projiziert, das einen aktiven Bereich abgrenzt, wobei der Operator ferner die physischen Parameter der Person, die behandelt werden soll, einstellt, wie zum Beispiel eine Dicke, Verarbeitungszeit pro Bereich, wodurch das Steuerungssystem durch Formeln, die im Hilfssystem gespeichert sind, eine Gesamtbehandlungszeit, eine Zahl von Bereichen, die behandelt werden sollen, und eine Sicherheitszeit für jeden Bereich neu berechnet, und wobei beim Abschalten der Vorrichtung das Steuerungssystem durch einen Punkt oder Fleck einen Bereich anzeigt, darauf den Ultraschallemittergriff anordnet und führt und das Steuerungssystem nach der Behandlung des Bereichs über eine eingestellte Behandlungszeit, die vom Hilfssystem eingestellt ist, den Ultraschallemitter abschaltet und den Punkt oder Fleck anzeigt und dann das Steuerungssystem einen zweiten Punkt anzeigt, der behandelt werden soll, während die Laserquelle und der Ultraschallemitter wieder eingeschaltet werden, wobei das offenbarte Verfahren bis zum Ende der Behandlung fortgesetzt ist.

## Revendications

1. Système de commande et de support (1) et appareil de traitement de taches sur la peau (2) comprenant une poignée de traitement,
- ledit système de commande et de support comprenant,
- une source laser (7) permettant d'émettre un faisceau laser et des moyens (4, 6, 11) permettant de commander et de positionner le faisceau laser de ladite source laser,
- des moyens (4) permettant de recevoir et de traiter, à partir desdits paramètres physiques déterminés par l'opérateur pour la personne devant être traitée, tel que l'épaisseur de la peau et la durée de traitement par région, à partir desquels lesdits moyens de traitement calculent, grâce à des formules mémorisées dans ledit système de commande et de support, un temps de traitement global, le nombre de régions devant être traitées, et une durée de sécurité, pour chaque région,
- ledit système de commande et de support (1) étant conçu pour que ladite source laser (7) et pour que lesdits moyens (4, 5, 6, 11) permettant de commander ledit faisceau laser, dans une opération active dudit appareil de traitement,
- projettent, sur une personne devant être traitée, un motif géométrique délimitant une région de traitement (10),
- indiquent une position au niveau de laquelle un opérateur doit positionner ladite poignée de traitement, et
- indiquent un temps de repos précis pendant lequel temps l'opérateur doit maintenir ladite poignée de traitement au niveau de l'endroit de traitement indiqué,
grâce à quoi, au moment de la mise en marche de l'appareil de traitement (2), le système de commande et de support (1) fait que lesdits moyens (4, 5, 6, 11) permettant de commander et de positionner le faisceau laser indiquent un point ou un spot, au niveau duquel ladite poignée de traitement doit être positionnée et, après que ledit temps de repos s'est écoulé, ledit système de commande éteigne ledit appareil de traitement (2) et ledit faisceau laser, et ensuite, ledit système de commande fait que ledit laser indique un second point ou un second spot devant être traité en remettant en marche ladite source laser (7) et ledit appareil de traitement (2), cette procédure se poursuivant jusqu'à la fin du temps de traitement.

2. Système de commande selon la revendication 1, **caractérisé en ce que** ledit système de commande (1) comprend un système auxiliaire conçu pour indiquer à l'opérateur, pendant le traitement, un temps de traitement approprié et un temps de repos précis au niveau d'une région de la peau déterminée.

3. Système de commande selon la revendication 1, **caractérisé en ce que** ledit système de commande (1) comprend une alimentation de réseau (3), fournissant une tension continue DC de +/- 24 V, un système de microprocesseur (4) permettant de recevoir et de traiter des données et de commander des galvanomètres (6) et une source laser (7), un tableau amplificateur de courant (5), permettant de commander chacun desdits galvanomètres, une source laser (7) et un capteur de distance (8).

4. Système de commande selon la revendications 1, **caractérisé en ce que** ledit système de commande (1) comprend une source laser à faible émission émettant un faisceau laser qui est réfléchi par deux miroirs (11) couplés auxdits galvanomètres de précision (6) correspondants, lesdits miroirs (11) étant montés sur des coordonnées X et Y et permettant de dévier le faisceau laser (12) généré par ladite source laser, avec une très grande vitesse, en dessinant ou en représentant ainsi n'importe quel type souhaité de motif sur la personne (9) devant être traitée tout en indiquant, pendant le traitement, un point ou un spot au niveau duquel l'opérateur doit agencer la poignée de l'appareil, lesdits galvanomètres (6) étant commandés par un système de microprocesseur (4) qui est connecté, par l'intermédiaire d'une porte de communication d'appareil de traitement, ledit appareil de traitement envoyant les données devant être utilisées par le système de commande vers les régions à motif devant être traitées et afin de positionner, en conséquence, la poignée de l'appareil.

5. Système de commande, selon la revendication 4, **caractérisé en ce que** ledit capteur de distance (8) permettant de détecter la distance entre la source laser (7) et la personne (9) devant être traitée, est de préférence constitué d'un capteur de distance à ultrasons (8), un capteur laser, un capteur IR, un capteur mécanique, pour permettre au système de commande de recalculer, de manière autonome, les paramètres de réflexion du laser afin de fournir toujours un positionnement et un dimensionnement constant ou uniforme de ladite poignée.

6. Système de commande selon la revendication 2, **caractérisé en ce que** ledit système auxiliaire (1) est déterminé par l'opérateur grâce à des paramètres physiques de la personne (9) devant être traitée, grâce à quoi un ordinateur, en se basant sur des formules préalablement entrées en mémoire, calcule les temps et les positions optimum permettant d'assurer un traitement efficace et sûr de ladite personne.

7. Système de commande selon la revendication 2, **caractérisé en ce que** ledit système auxiliaire, en coopération avec ledit système de commande (1), permet, en déterminant lesdits paramètres physiques de la personne (9) devant être traitée, d'indiquer un temps de traitement global approprié, un positionnement de la poignée de l'appareil au niveau de la région de la peau (10) devant être traitée, et un temps de repos de ladite poignée pour chaque région de peau devant être traitée, ledit système auxiliaire envoyant ces informations au système de commande (1) en affichant tour à tour précisément sur la personne (9) devant être traitée le temps de fonctionnement et le positionnement de la poignée de l'appareil devant être respectés par l'opérateur.

8. Système de commande selon la revendication 2, **caractérisé en ce que**, avec ledit système de commande appliqué à un appareil de traitement par cavitation, en coopération avec ledit système auxiliaire, ledit opérateur détermine, à partir dudit appareil par cavitation, une région de la peau devant être traitée, grâce à quoi ledit système de commande projettera sur la personne devant être traitée un motif géométrique délimitant une région active, l'opérateur déterminant en outre lesdits paramètres physiques de la personne devant être traitée, tel qu'une épaisseur, un temps de traitement par région, grâce à quoi ledit système de commande va recalculer, grâce à des formules entrées en mémoire dans ledit système auxiliaire, un temps de traitement global, un nombre de régions devant être traitées et un temps de sécurité, pour chaque région, et, au moment de l'arrêt de l'appareil, ledit système de commande indiquant par un point ou un spot une région au niveau de laquelle agencer et diriger la poignée émettrice d'ultrasons, et, après avoir traité ladite région pendant un temps de traitement déterminé par ledit système auxiliaire, ledit système de commande arrêtant l'émetteur d'ultrasons et indiquant le point ou le spot, et ensuite le système de commande indiquant un second point devant être traité tout en remettant en marche ladite source laser et ledit émetteur d'ultrasons, la procédure décrite se poursuivant jusqu'à la fin du traitement.
